**Europäisches Patentamt**

**European Patent Office**

**Office européen des brevets**

(19)

(11) Veröffentlichungsnummer: **0 006 523**
**B1**

(12) EUROPÄISCHE PATENTSCHRIFT

(45) Veröffentlichungstag der Patentschrift: **22.07.81**

(51) Int. Cl.³: **C 07 C 47/07**, C 07 C 45/34

(21) Anmeldenummer: **79101879.9**

(22) Anmeldetag: **11.06.79**

(54) **Verfahren zur Herstellung von Acetaldehyd.**

(30) Priorität: **22.06.78 DE 2827380**

(43) Veröffentlichungstag der Anmeldung:
**09.01.80 Patentblatt 80/1**

(45) Bekanntmachung des Hinweises auf die Patenterteilung:
**22.07.81 Patentblatt 81/29**

(84) Benannte Vertragsstaaten:
**CH DE FR IT**

(56) Entgegenhaltungen:
**DE-A-2 612 797**
**DE-B-1 149 704**

(73) Patentinhaber: **HOECHST AKTIENGESELLSCHAFT,**
**Postfach 80 03 20, D-6230 Frankfurt/Main 80 (DE)**

(72) Erfinder: **Steppich, Walter, Dr., Am Hohen Stein 14,**
**D-6200 Wiesbaden (DE)**
Erfinder: **Sartorius, Rudolf, Dr., Am Leisrain 2,**
**D-6230 Frankfurt am Main 80 (DE)**

BUNDESDRUCKEREI BERLIN

# 0 006 523

### Verfahren zur Herstellung von Acetaldehyd

Die einstufige Herstellung von Acetaldehyd durch Direktoxidation von Ethylen in Gegenwart von Palladiumchlorid und Wasser ist bereits bekannt (Jira, Blau, Grimm; Hydrocarbon Processing, März 1976, S. 97 – 100). Bei der technischen Durchführung geht man im allgemeinen wie folgt vor· Ethylen wird in einem Blasensäulenreaktor, der eine wäßrige Lösung von $CuCl_2$, $CuCl$ und $PdCl_2$ enthält, mit Sauerstoff in einem Kreislaufverfahren bei 100 bis 150°C und 1 bis 6 bar (absoluter Druck), vorzugsweise bei 125 bis 135°C und 3 bis 4,5 bar (absoluter Druck), zu Acetaldehyd oxidiert. Der Ausdruck »einstufige Herstellung« bedeutet dabei, daß die Oxidation des Ethylens zu Acetaldehyd und die Reoxidation des dabei reduzierten Palladiumchlorids (durch $CuCl_2$, welches dabei in $CuCl$ übergeht, das seinerseits durch den Sauerstoff reoxidiert wird) im selben Reaktor durchgeführt wird. Der den Reaktor verlassende Gasstrom, der Wasserdampf, Acetaldehyd, Ethylen sowie geringe Mengen Sauerstoff, Kohlendioxid, Essigsäure, Crotonaldehyd und chlorierte Verbindungen (wie Methylchlorid, Ethylchlorid und Chloracetaldehyde) enthält, wird in einem Kondensator zunächst auf etwa 80 bis 130°C, vorzugsweise 105 bis 115°C, abgekühlt. Das dabei gebildete Kondensat, das im wesentlichen aus Wasser sowie kleinen Mengen Acetaldehyd und Essigsäure besteht, wird im allgemeinen in den Reaktor zurückgeführt.

Während die flüchtigen Nebenprodukte mit dem Acetaldehyd und den unumgesetzten Ausgangsstoffen den Reaktor verlassen, verbleiben ebenfalls gebildete geringe Mengen an Kupferoxalat und hochmolekularen Nebenprodukten in der Katalysatorlösung. Um deren Anreicherung zu verhindern, wird ständig ein kleiner Teil der flüssigen Phase aus dem Reaktor abgezogen. Dann wird dieser Teil zunächst entspannt, wobei die gelösten Leichtsieder, wie Acetaldehyd, Ethylen und Kohlendioxid in die Gasphase übergehen und entfernt werden. Die entgaste Lösung wird in ein Regenerationsgefäß geleitet und dort − z. B. mit Hilfe von eingeleitetem Wasserdampf (»Direktdampf«) − auf etwa 165 bis 180°C erhitzt, wobei Kupferoxalat und die hochmolekularen Nebenprodukte unter Bildung von Wasser und Kohlendioxid zersetzt werden. Die regenerierte Lösung wird in den Reaktor zurückgeführt.

Nachdem der den Reaktor verlassende Gasstrom bereits im Kondensator abgekühlt worden ist, wird er im allg. in Wärmetauschern weiter auf etwa 30 bis 80°C, vorzugsweise 40 bis 50°C, abgekühlt. Dann wird der Acetaldehyd mit Wasser in einem Waschturm aus dem Gasstrom ausgewaschen. Das verbleibende Restgas, das vorwiegend aus Ethylen, Sauerstoff, Kohlendioxid und Inertgasen besteht, wird nach Ausschleusung einer Teilmenge (um Anreicherung von Kohlendioxid und Inertgasen zu vermeiden) und nach Zugabe von frischem Ethylen wieder dem Reaktor zugeführt. Das Kondensat aus den Wärmetauschern und die im Waschturm entstandene wäßrige Acetaldehyd-Lösung werden in einem Sammelbehälter vereinigt. Diese »Rohaldehyd« genannte Mischung wird von dort einer zweistufigen Destillation zugeführt.

Hierbei werden in der 1. Destillationsstufe in einer Extraktivdestillation mit Wasser als Extraktionsmittel die Leichtsieder (Methylchlorid, Ethylchlorid) und die gelösten Gase, vorwiegend Ethylen ud Kohlendioxid, über Kopf destilliert. Das Sumpfprodukt wird der 2. Destillationsstufe zugeführt. In dieser wird über Kopf reiner Acetaldehyd gewonnen. Mittels eines Seitenabzuges wird eine vorwiegend Crotonaldehyd enthaltende Fraktion abgenommen. Die hochsiedenden Nebenprodukte (vor allem Essigsäure und Chloracetaldehyde) werden mit dem Wasser aus dem Sumpf abgezogen. Das abgezogene Gemisch wird im folgenden als »Abwasser« bezeichnet.

Der Wärmebedarf beider Destillationsstufen wird im allgemeinen durch Einspeisung von Wasserdampf in deren Sümpfe gedeckt (»Direktdampf-Heizung«). Hierzu werden pro Tonne Acetaldehyd etwa 1,3 Tonnen Direktdampf verbraucht.

Damit die oben erwähnte Auswaschung des Acetaldehyds möglichst vollständig ist, müssen große Mengen Wasser eingesetzt werden. Die Acetaldehyd-Konzentration in der dabei entstehenden, den Hauptteil des Rohaldehyds bildenden, wäßrigen Lösung liegt aus diesem Grunde relativ niedrig. Entsprechend hohe Mengen Abwassers fallen am Sumpf der 2. Destillationsstufe an, wobei das Abwasser neben dem erwähnten Waschwasser den kondensierten Wasserdampf aus der Direktdampfheizung beider Destillationsstufen enthält. Pro Tonne Acetaldehyd fallen etwa 8 bis 10 m³ Abwasser an, wovon 1,3 m³ aus dem Direktdampf entstehen. Das Abwasser enthält Essigsäure, Carbonylverbindungen (vorwiegend Kondensationsprodukte des Acetaldehyds und Chloracetaldehyd) sowie chlorierte organische Verbindungen (vorwiegend Chloracetaldehyde) und muß daher, um eine Umweltbelastung zu vermeiden, einer Nachbehandlung unterworfen werden. Es ist zwar biologisch abbaubar, aber dies erfordert aufgrund der Menge und des hohen CSB-Wertes einen erheblichen Aufwand.

Überraschenderweise ist es nun möglich, das Abwasser im Kreis zu führen. Das bedeutet, daß man vor allem das Auswaschen des Acetaldehyds aus dem Gasstrom mit Abwasser vornehmen kann. Außerdem kann man die Verdampfungsverluste der wäßrigen Katalysatorlösung − Wasser wird mit den Reaktionsprodukten dauernd aus dem Reaktor ausgetragen − durch Abwasser ersetzen, wofür bisher vollentsalztes Wasser erforderlich war. Schließlich kann auch für die Extraktivdestillation in der 1. Destillationskolonne ohne weiteres Abwasser benutzt werden.

2

Ein Teilstrom des Abwassers wird laufend ausgeschleust. Dadurch wird erreicht, daß sich die Verunreinigungen in dem zurückgeführten Abwasser nur bis zu einem Gleichgewichtszustand anreichern können.

Das erfindungsgemäße Verfahren zur Herstellung von Acetaldehyd durch Umsetzung von Ethylen und Sauerstoff in Gegenwart einer wäßrigen Lösung von Palladium- und Kupferchlorid, wobei dauernd ein Teil dieser Lösung außerhalb des Reaktors durch Erhitzen regeneriert wird und die Aufarbeitung des den Reaktor verlassenden gasförmigen Gemisches durch a) Abkühlen unter Rückführung des dabei gebildeten Kondensats in den Reaktor und Ergänzung des beim Abkühlen nicht kondensierten Wasseranteils durch Zufuhr von Wasser in den Reaktor, b) Waschen des abgekühlten Gasgemisches mit Wasser unter Gewinnung einer wäßrigen Acetaldehydlösung, c) Extraktivdestillation dieser Lösung mit Wasser als Extraktionsmittel und d) Gewinnung von reinem Acetaldehyd als Kopfprodukt in einer weiteren Destillationsstufe erfolgt, ist dadurch gekennzeichnet, daß man mit dem am Sumpf der letztgenannten Destillationsstufe anfallenden Abwasser den Wasserbedarf der Stufe b) und ggf. zusätzlich den Wasserbedarf der Stufen a) und/oder c) deckt und den Rest des Abwassers ausschleust.

Auf jeden Fall wird also die Wasserwäsche des Gasgemisches zur Gewinnung der wäßrigen Acetaldehydlösung erfindungsgemäß mit dem Abwasser vorgenommen. Vorteilhafterweise wird zusätzlich 1) auch das aus dem Reaktor gasförmig ausgetragene und beim Abkühlen nicht kondensierte Wasser durch Einleitung von Abwasser in den Reaktor ersetzt oder 2) der Wasserbedarf der Extraktivdestillation (Schritt c) mit Abwasser gedeckt. Besonders bevorzugt ist es, zusätzlich zur Acetaldehyd-Auswaschung mit Abwasser sowohl Maßnahme 1) als auch Maßnahme 2) durchzuführen.

Vor der Rückführung wird das Abwasser auf Normaltemperatur abgekühlt. Die Menge des ausgeschleusten Abwassers entspricht natürlich der Menge des von außen eingeführten Wassers. Das heißt, wenn in Schritt a) und/oder c) Frischwasser benutzt wird, muß eine entsprechende Abwassermenge ausgeschleust werden. Wenn zur Beheizung der Destillationsstufen c) und/oder d) Direktdampf benutzt wird, muß ebenfalls eine entsprechende Menge Abwasser ausgeschleust werden. Wenn man, was bevorzugt ist, beide Destillationsstufen c) und d) mit Direktdampf beheizt und Abwasser sowohl in Schritt b) als auch in Schritt a) und Schritt c) zurückführt, so ist lediglich das durch den Direktdampf eingeführte Wasser wieder auszuschleusen. Das ausgeschleuste Wasser wird im allgemeinen biologisch aufgearbeitet.

Von der Kreisführung des Abwassers abgesehen geht man so vor, wie zu Beginn beschrieben, insbesondere was die angegebene Temperaturbereiche betrifft. Daß das Wasser im Kreis geführt werden kann, ist überraschend, weil wegen seines Gehalts an Verunreinigungen unvollständige Auswaschung des Acetaldehyd und Störungen im Reaktor (Bildung unlöslicher Rückstände und Katalysatorschädigung) zu befürchten waren.

Es war außerdem zu erwarten, daß bei Rückführung des Abwassers nach wie vor 1,3 Tonnen Direktdampf pro Tonne Acetaldehyd für die beiden Destillationsstufen benötigt würden. Dies ist überraschenderweise nicht der Fall. Es wird für die Auswaschung des Acetaldehyds weniger Wasser benötigt, wenn mit rückgeführtem Abwasser (im folgenden »Rückwasser« genannt) statt mit Frischwasser gearbeitet wird. An sich war anzunehmen, daß man wegen der Verunreinigung des Rückwassers dieses in größerer Menge bei der Wäsche benötigen würde. Statt dessen ist der Verbrauch um ca. 20% niedriger als bei der Verwendung von Frischwasser. Daher sinkt auch der Bedarf an Direktdampf für die zweistufige Destillation des (nunmehr weniger stark verdünnten) Rohaldehyds um annähernd 20%. Des weiteren war zu erwarten, daß die im Rückwasser enthaltenen Chloracetaldehyde unter den Reaktionsbedingungen gespalten würden und dadurch die Konzentration an Chlorionen ansteigen und dies wegen der ebenfalls im Rückwasser enthaltenen Essigsäure zu Korrosionsproblemen in der Anlage führen würde. Die durchgeführten Versuche haben aber gezeigt, daß die Spaltung der Chloracetaldehyde nur in einem unwesentlichen Umfang erfolgt. Der für solche Anlagen üblicherweise benutzte Chrom-Nickel-Edelstahl entspricht bei sachgerechter Verarbeitung auch den erhöhten Anforderungen und eine Rückführung des Abwassers kann ohne Änderung des Werkstoffs der Kolonnen, also auch in vorhandenen Anlagen, durchgeführt werden.

Eine weitere überraschende Feststellung ergibt sich bei der Bestimmung der in dem ausgeschleusten Teil des Abwassers enthaltenen Nebenprodukte. Es war anzunehmen, daß bei der erfindungsgemäßen Rückführung des Abwassers dieselbe Menge an Verunreinigungen gebildet würde, wie bei Verwendung von Frischwasser gemäß dem Stand der Technik. Die Analyse der Verunreinigungen in dem ausgeschleusten Abwasserteilstrom ergibt jedoch kleinere Mengen als sie im Abwasser bei Verwendung von Frischwasser gefunden werden.

Der ausgeschleuste Abwasserteilstrom läßt sich überraschenderweise leicht destillativ aufarbeiten. Es wurde nämlich gefunden, daß die in diesem Abwasser enthaltenen Verunreinigungen (außer Essigsäure) als Kopfprodukt einer entsprechend dimensionierten Kolonne weitgehend abgetrennt werden können, wobei im Sumpf der Kolonne ein im wesentlichen nur noch Essigsäure enthaltendes Wasser anfällt, wodurch die biologische Aufarbeitung entlastet wird. In Anbetracht der sehr niedrigen Siededifferenz zwischen Sumpf- und Kopfprodukt von nur etwa 2°C war dies ein überraschender Befund. Das Kopfprodukt führt man vorzugsweise in die Katalysator-Regeneration ein, in der es

3

vollständig zu Wasser, Kohlendioxid und Salzsäure abgebaut wird. Zur Vermeidung von Mißverständnissen ist zu beachten, daß »Abwasser« definitionsgemäß das Sumpfprodukt der 2. Destillationskolonne ist. Das »ausgeschleuste« Abwasser ist der Teil des Abwassers, der entweder vollständig der biologischen Aufarbeitung zugeführt wird oder von dem nur eine relativ kleine (verglichen mit dem Sumpfprodukt) Kopffraktion zurückgeführt wird. Im letztgenannten Fall wird das »ausgeschleuste Abwasser« nicht zurückgeführt, wohl aber eine Fraktion davon.

Vergleichsbeispiel

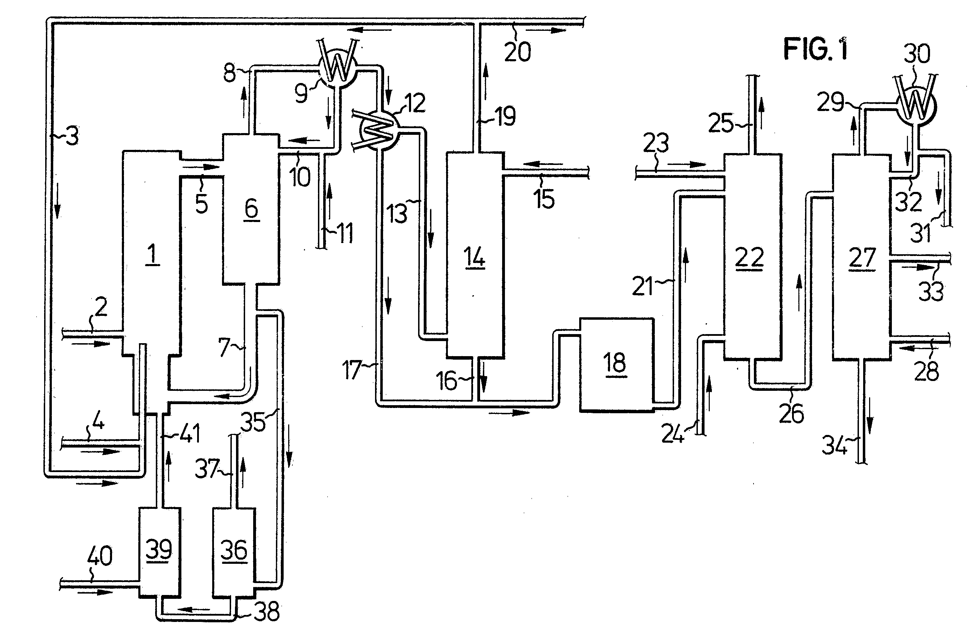

a) Apparativer Aufbau (vgl. Figur 1)

Im Reaktor (1) befindet sich eine wäßrige Lösung von $CuCl_2$, CuCl und $PdCl_2$. Über Leitung (2) wird Sauerstoff zugeführt und über Leitung (3) Kreisgas, dem über Leitung (4) frisches Ethylen beigemischt wird. Das im Reaktor entstehende Gas-Flüsigkeits-Gemisch aus gasförmigen Ausgangs- und Endprodukten sowie Katalysatorlösung gelangt über das Rohr (5) in den Abscheider (6), wo sich Gasphase und Flüssigkeitsphase trennen. Die Flüssigkeit gelangt über Leitung (7) zurück in den Reaktor (1). Die Gase verlassen den Abscheider über Leitung (8) und werden im Vorkondensator (9) auf 110°C abgekühlt. Das dabei gebildete Kondensat wird über Leitung (10) — nach Beimischung von entsalztem Wasser zum Ersatz des mit den Reaktionsprodukten aus dem Reaktor ausgetragenen Wassers der Katalysatorlösung über Leitung (11) — in den Abscheider und von dort über Leitung (7) in den Reaktor zurückgeführt. Die im Vorkondensator (9) noch nicht kondensierten Gase werden im Wärmetauscher (12) weiter abgekühlt und über Leitung (13) in den Waschturm (14) überführt. Mit über Leitung (15) zugeführtem Wasser wird hier der Acetaldehyd aus den Gasen gewaschen. Die entstehende Acetaldehyd-Lösung gelangt über Leitung (16) und das im Wärmetauscher (12) entstandene Kondensat über Leitung (17) in den Sammelbehälter (18). Die gewaschenen Gase verlassen den Waschturm am Kopf und werden über Leitung (19) als Kreisgas zum Reaktor (1) zurückgeführt, nachdem eine Teilmenge über Leitung (20) als Abgas ausgeschleust worden ist. Die als »Rohaldehyd« bezeichnete Mischung wird aus dem Behälter (18) über Leitung (21) der 1. Destillationskolonne (22) zugeführt und dort einer Extraktivdestillation mit Wasser unterworfen. Das hierzu benötigte Wasser wird über Leitung (23) zugeführt. Die Kolonne wird durch Dampf geheizt, der über Leitung (24) eingespeist wird. Das Kopfprodukt (im wesentlichen Methylchlorid, Ethylchlorid, Kohlendioxid und Ethylen) wird über Leitung (25) abgezogen. Das Sumpfprodukt wird über Leitung (26) der 2. Destillationskolonne (27) zugeführt, die wiederum mit Direktdampf geheizt wird, der über Leitung (28) eingespeist wird. Über Leitung (29) wird reiner Acetaldehyd als Kopfprodukt abgezogen. Nach Kondensation im Wärmetauscher (30) wird die Hauptmenge über Leitung (31) abgezogen und eine Teilmenge über Leitung (32) als Rücklauf in die Kolonne (27) zurückgeführt. Über einen Seitenabzug (33) wird eine im wesentlichen aus Crotonaldehyd bestehende Fraktion abgezogen. Die hochsiedenden Nebenprodukte (vor allem Essigsäure und Chloracetaldehyde) und das Wasser werden über Leitung (34) als »Abwasser« am Sumpf abgezogen. Aus der über Leitung (7) vom Abscheider (6) in den Reaktor (1) zurückfließende Flüssigkeit wird über Leitung (35) laufend eine Teilmenge ausgeschleust und im Entspannungsgefäß (36) entspannt. Dabei gehen die gelösten Leichtsieder in die Gasphase über und werden über Leitung (37) entfernt. Die entgaste Lösung wird über Leitung (38) in den Regenerator (39) geleitet und dort mit Hilfe von über Leitung (40) eingeleitetem Dampf auf 170°C erhitzt. Anschließend wird die Lösung über Leitung (41) in den Reaktor (1) zurückgeführt.

b) Versuchsdurchführung

In der beschriebenen Anlage werden pro Stunde aus 5,3 t Sauerstoff und 9 t Ethylen in Gegenwart einer Katalysatorlösung, die aus 100 t Wasser, 100 kMol CuCl und $CuCl_2$ sowie 60 kg $PdCl_2$ besteht, 13,0 t Acetaldehyd hergestellt. Dabei werden folgende Mengen an Wasser und Dampf verbraucht:

| | |
|---|---|
| 15 m³/h | vollentsalztes Wasser für den Reaktor (Ersatz der Verdampfungsverluste), zugeführt über Leitung (11) |
| 77,5 m³/h | Fluß- oder Brunnenwasser zum Auswaschen des Acetaldehyds, zugeführt über Leitung (15) |
| 1,5 t/h | Direktdampf für die Katalysator-Regenerierung, zugeführt über Leitung (40) |
| 1,5 m³/h | vollentsalztes Wasser für die Extraktion in der 1. Destillationskolonne, zugeführt über Leitung (23) |
| 3 t/h | Direktdampf für die 1. Destillationskolonne, zugeführt über Leitung (24) |
| 14 t/h | Direktdampf für die 2. Destillationskolonne, zugeführt über Leitung (28) |

Aus diesem Wasser- und Dampfeinsatz resultiert eine Abwassermenge von 112,5 m³/h, die als Sumpfprodukt der Kolonne (27) anfällt.

4

Dieses Abwasser enthält:

| | | |
|---|---|---|
| 0,28% | Säuren (gerechnet als Essigsäure) | = 315 kg/h |
| 0,036% | Aldehyde (gerechnet als Acetaldehyd) | = 45 kg/h |
| 200 ppm | organisch gebundenes Chlor (gerechnet als Chloracetaldehyd) | = 49,8 kg/h |
| 2700 mg | $O_2$/l CSB, d. h. $O_2$-Bedarf | = 304 kg/h |

Das Abwasser wird biologisch aufgearbeitet.

## Beispiel 1

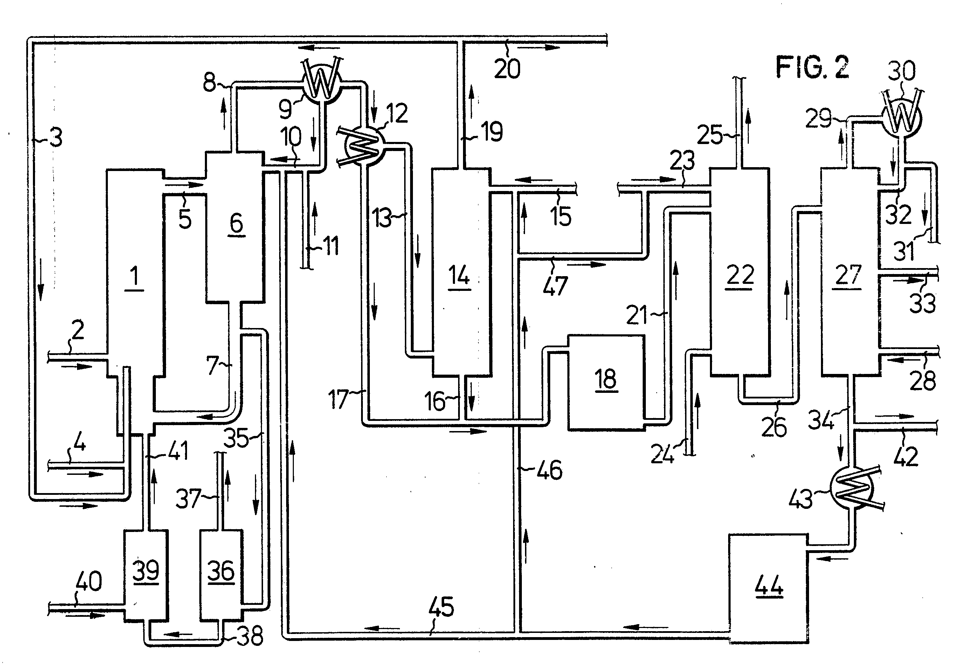

### a) Apparativer Aufbau (vgl. Figur 2)

Die Apparatur ist aufgebaut wie im Vergleichsbeispiel, jedoch mit folgender Ausnahme: Das am Sumpf der 2. Destillationskolonne (27) über Leitung (34) abgezogene Abwasser wird — nach Ausschleusung einer Teilmenge über Leitung (42) — über den Kühler (43) in den Vorratsbehälter (44) geleitet und von dort in den Prozeß zurückgeführt:

1. In den Abscheider (6) über Leitung (45) und Leitung (10) zum Ersatz des verdampften Wassers der Katalysatorlösung (das im Vergleichsbeispiel durch Zufuhr von vollentsalztem Wasser über Leitung (11) ersetzt wurde).
2. In den Waschturm (14) über die von Leitung (45) abzweigende Leitung (46), die in Leitung (15) einmündet (über die im Vergleichsbeispiel Frischwasser zugeführt wurde, was jetzt nur noch zu Beginn des Prozesses erforderlich ist).
3. In die 1. Destillationskolonne (22) über die von Leitung (46) abzweigende Leitung (47), die in Leitung (23) einmündet (über die im Vergleichsbeispiel Frischwasser zugeführt wurde, was jetzt ebenfalls nur noch zu Beginn des Prozesses erforderlich ist).

### b) Versuchsdurchführung

Es werden wie im Vergleichsbeispiel pro Stunde 13,0 Tonnen Acetaldehyd hergestellt. Der einzige Unterschied besteht in der Rückführung der Hauptmenge des Abwassers.

Nach Einstellung stationärer Bedingungen werden 15,7 m³/h = 16,6% des anfallenden Abwassers über Leitung (42) ausgeschleust. Diese Menge entspricht dem aus dem Direktdampf entstandenen Kondensat.

Der größere Teil, nämlich 79 m³/h, d. h. 83,3% des anfallenden Abwassers wird auf 15 bis 20°C abgekühlt und als »Rückwasser« zurückgeführt.

Es ergibt sich folgende Wasserbilanz:

| | |
|---|---|
| 15 m³/h | Rückwasser zum Reaktor (Ersatz der Verdampfungsverluste), zugeführt über Leitung (45) |
| 62,5 m³/h | Rückwasser zum Auswaschen des Acetaldehyds, zugeführt über Leitung (46) |
| 1,5 t/h | Direktdampf für die Katalysator-Regeneration, zugeführt über Leitung (40) |
| 1,5 m³/h | Rückwasser für die Extraktion in der 1. Destillationskolonne, zugeführt über Leitung (47) |
| 1,7 t/h | Direktdampf für die 1. Destillationskolonne, zugeführt über Leitung (24) |
| 12,5 t/h | Direktdampf für die 2. Destillationskolonne, zugeführt über Leitung (28) |

Insgesamt ergibt sich daraus ein Abwasseranfall von 94,7 m³/h.

Das über Leitung (42) ausgeschleuste Abwasser enthält:

| | | |
|---|---|---|
| 1,7% | Säuren (gerechnet als Essigsäure) | = 267 kg/h |
| 0,24% | Aldehyde (gerechnet als Acetaldehyd) | = 38 kg/h |
| 700 ppm | organisch gebundenes Chlor (gerechnet als Chloracetaldehyd) | = 24,3 kg/h |
| 18 000 mg | $O_2$/l CSB, d. h. $O_2$-Bedarf | = 283 kg/h |

Das ausgeschleuste Abwasser wird biologisch aufgearbeitet.

## Beispiel 2

### Destillative Aufarbeitung des ausgeschleusten Abwasserteilstroms

Man verfährt wie in Beispiel 1, jedoch wird der über Leitung (42) ausgeschleuste Abwasserteilstrom in eine Kolonne mit 45 Glockenböden eingespeist.

5

Der Einlauf befindet sich auf dem 30. Boden (von unten gerechnet). Bei einem Rücklaufverhältnis von 1 : 3, einer Kopftemperatur von 98°C und einer Sumpftemperatur von 100°C werden 2,79% der eingespeisten Menge als Destillat am Kopf entnommen.

Pro Liter Einlauf werden somit 27,9 g Kopfprodukt und 972,ï g Sumpfprodukt erhalten.

Das Sumpfprodukt enthält neben Wasser:

| | |
|---|---|
| 1,75% | Säuren (gerechnet als Essigsäure) |
| 0,08% | Aldehyde (gerechnet als Acetaldehyd) |
| 135 ppm | organisch gebundenes Chlor |
| | = 0,03% (gerechnet als Chloracetaldehyd) |
| 15 400 mg | $O_2$/l CSB |

Das Kopfprodukt enthält neben Wasser:

| | |
|---|---|
| 0,05% | Säuren (gerechnet als Essigsäure) |
| 6,34% | Aldehyde (gerechnet als Acetaldehyd) |
| 1,7% | organisch gebundenes Chlor |
| | = 3,76% (gerechnet als Chloracetaldehyd) |

Bei einer Produktion von 13,0 t/h Acetaldehyd ergeben sich daher folgende Mengen:

| | |
|---|---|
| Sumpfprodukt der destillativen Aufarbeitung des ausgeschleusten Abwasserteilstroms | 15,3 m³/h |
| Kopfprodukt der destillativen Aufarbeitung des ausgeschleusten Abwasserteilstroms | 438 kg/h |

In diesem Sumpfprodukt sind enthalten (außer Wasser):

| | |
|---|---|
| Säuren (gerechnet als Essigsäure) | 268 kg/h |
| Aldehyde (gerechnet als Acetaldehyd) | 12,2 kg/h |
| Organisch gebundenes Chlor (gerechnet als Chloracetaldehyd) | 4,6 kg/h |
| $O_2$-Bedarf (CSB) | 236 kg/h |

Das Sumpfprodukt wird biologisch aufgearbeitet.

Im Kopfprodukt der Aufarbeitungskolonne sind enthalten:

| | |
|---|---|
| Säuren | Spuren |
| Aldehyde (gerechnet als Acetaldehyd) | 27,6 kg/h |
| Organisch gebundenes Chlor (gerechnet als Chloracetaldehyd) | 16,5 kg/h |
| Wasser | 394 kg/h |

Das Kopfprodukt wird kontinuierlich in den Regenerator (39) eingespeist, wo es unter den Betriebsbedingungen vollständig zu $CO_2$, Wasser und HCl abgebaut wird.

Es ergibt sich folgende Wasserbilanz:

| | |
|---|---|
| 14,6 m³/h | Rückwasser zum Reaktor |
| 0,4 m³/h | Kopfprodukt der Abwasserdestillation (über Regenerator zum Reaktor) |
| 62,5 m³/h | Rückwasser zum Auswaschen des Acetaldehyds |
| 1,5 t/h | Direktdampf für die Katalysator-Regeneration |
| 1,5 t/h | Rückwasser für die Extraktion in der 1. Destillationskolonne |
| 1,7 t/h | Direktdampf für die 1. Destillationskolonne |
| 12,5 t/h | Direktdampf für die 2. Destillationskolonne |

Insgesamt werden also wie in Beispiel 1 79 m³/h Abwasser (Rückwasser) im Kreislauf geführt. Als vorgereinigtes Abwasser fallen dabei 15,3 m³/h an.

Bei einem Vergleich des Säurewertes mit dem CSB-Wert ist anzumerken, daß der CSB-Wert unter dem aus dem Säuregehalt theoretisch errechneten liegt (berechnet 272 kg/h, gefunden 235 kg/h).

Dies liegt darin begründet, daß bei der titrimetrischen Bestimmung der Essigsäure andere, nicht oxidable Säuren, bzw. weniger Sauerstoff verbrauchende Säuren, wie z. B. Ameisensäure, mit erfaßt werden.

Die Beheizung der Aufarbeitungskolonne kann selbstverständlich auch mit Direktdampf erfolgen, wobei sich der Wasseranfall um die Menge des kondensierenden Direktdampfes erhöht.

| | Art des zugeführ-ten Wassers | Zweck der Wasserzufuhr | Einheit | Vergleichs-beispiel | Beispiel 1 | Beispiel 2 |
|---|---|---|---|---|---|---|
| Reaktor (1) | a) vollentsalztes Wasser | Ersatz der Ver-dampfungsverluste | m³/h | 15,0 | — | — |
| | b) Rückwasser | | m³/h | — | 15,0 | 14,6 |
| | c) Kopfprodukt der Abwasser-destill. | | m³/h | — | — | 0,4 |
| Wasch-turm (14) | a) Frischwasser | Auswaschen des Acetaldehyds | m³/h | 77,5 | — | — |
| | b) Rückwasser | | m³/h | — | 62,5 | 62,5 |
| 1. Destill.-kolonne (22) | a) vollentsalztes Wasser | Extraktion | m³/h | 1,5 | — | — |
| | b) Rückwasser | | m³/h | — | 1,5 | 1,5 |
| | c) Dampf | Kolonnenheizung | t/h | 3,0 | 1,7 | 1,7 |
| 2. Destill.-kolonne (27) | Dampf | Kolonnenheizung | t/h | 14,0 | 12,5 | 12,5 |
| Regenera-tor (39) | Dampf | Katalysator-Regeneration | t/h | 1,5 | 1,5 | 1,5 |
| Biologisch aufzube-reitendes Wasser | Menge | | m³/h | 112,5 | 15,7 | 15,3 |
| | CSB | | kg $O_2$/h | 304 | 283 | 236 |
| | Säuren (gerechnet als Essigsäure) | | kg/h | 315 | 267 | 267 |
| | Aldehyde (gerechnet als Acetaldehyd) | | kg/h | 45 | 38 | 12,2 |
| | Organisch gebundenes Chlor (gerechnet als Chloracetaldehyd) | | kg/h | 49,7 | 24,3 | 4,6 |

**Patentansprüche**

1. Verfahren zur Herstellung von Acetaldehyd durch Umsetzung von Ethylen und Sauerstoff in Gegenwart einer wäßrigen Lösung von Palladium- und Kupferchlorid, wobei dauernd ein Teil dieser Lösung außerhalb des Reaktors durch Erhitzen regeneriert wird und die Aufarbeitung des den Reaktor verlassenden gasförmigen Gemisches durch a) Abkühlen unter Rückführung des dabei gebildeten Kondensats in den Reaktor und Ergänzung des beim Abkühlen nicht kondensierten Wasseranteils durch Zufuhr von Wasser in den Reaktor, b) Waschen des abgekühlten Gasgemisches mit Wasser unter Gewinnung einer wäßrigen Acetaldehydlösung, c) Extraktivdestillation dieser Lösung mit Wasser als Extraktionsmittel und d) Gewinnung von reinem Acetaldehyd als Kopfprodukt in einer weiteren Destillationsstufe erfolgt, dadurch gekennzeichnet, daß man mit dem am Sumpf der letztgenannten Destillationsstufe anfallenden Abwasser den Wasserbedarf der Stufe b) und gegebenenfalls zusätzlich den Wasserbedarf der Stufen a) und/oder c) deckt und den Rest des Abwassers ausschleust.

2. Verfahren nach Anspruch 1, dadurch gekennzeichnet, daß durch Destillation des ausgeschleusten Abwassers ein flüchtige Verunreinigungen enthaltendes Kopfprodukt gewonnen wird, welches in die

**0 006 523**

Regeneration eingespeist wird und als Sumpfprodukt ein im wesentlichen nur noch Essigsäure enthaltendes Wasser anfällt.

## Claims

1. Process for the manufacture of acetaldehyde by reaction of ethylene and oxygen in the presence of an aqueous solution of palladium and copper chloride, in which continuously part of the latter solution is regenerated by heating it outside of the reactor and in which the gaseous mixture leaving the reactor is worked up a) by cooling with recycling of the condensate formed to the reactor and completion of the water portion that has not condensed during cooling, by the addition of water to the reactor, b) by washing of the cooled gas mixture with water whereby an aqueous acetaldehyde solution is obtained, c) by extractive distillation of this solution using water as extraction agent and d) in which pure acetaldehyde is obtained as head product in a further distillation step, characterized by satisfying the water demand of step b) and optionally additionally of steps a) and/or c) with the waste water obtained in the sump of the last distillation step and discharging the residual waste water.

2. The process as claimed in claim 1, characterized by obtaining by distillation of the waste water discharged a head product containing volatile impurities, which is fed to the regeneration step and as sump product water containing substantially only acetic acid.

## Revendications

1. Procédé de préparation de l'acétaldéhyde par réaction de l'éthylène avec l'oxygène en présence d'une solution aqueuse de chlorure de palladium et de chlorure de cuivre, une partie de cette solution étant constamment régénérée par chauffage en dehors du réacteur et le traitement complémentaire du mélange gazeux sortant du réacteur comprenant les opérations suivantes:

a) refroidissement, avec renvoi au réacteur du produit de condensation ainsi formé et apport d'eau au réacteur pour remplacer celle qui n'a pas été condensée lors du refroidissement,

b) lavage à l'eau du mélange gazeux refroidi, fournissant une solution aqueuse d'acétaldéhyde,

c) distillation extractive de cette solution avec de l'eau comme agent d'extraction et

d) isolement d'acétaldéhyde pur comme produit de tête dans une autre étape de distillation, caractérisé en ce qu'on couvre les besoins en eau de l'étape b) et éventuellement ceux des étapes a) et/ou c) avec l'eau usée formée dans le bouilleur de la dernière étape de distillation mentionnée, et on fait sortir le reste de l'eau usée.

2. Procédé selon la revendication 1, caractérisé en ce que, par distillation de l'eau usée retirée, on isole un produit de tête contenant des impuretés volatiles, produit que l'on introduit dans la régénération, et, comme produit du bouilleur, une eau ne contenant pratiquement plus que de l'acide acétique.

8

FIG.1

FIG. 2